# EUROPEAN PATENT APPLICATION

(11) **EP 1 110 525 A1**
(43) Date of publication of application: **27.06.2001**
(21) Application number: 99125936.7
(22) Date of filing: 23.12.1999
(51) Int. Cl.: A61F 13/15, A61F 5/455

(54) **Liquid handling members with membrane regions having varying surface properties**

(71) Applicant: THE PROCTER & GAMBLE COMPANY, Cincinnati, Ohio 45202 (US)
(72) Inventor: Schmidt, Matthias, 65510 Idstein (DE); Ehrnsperger, Bruno Johannes, 65936 Frankfurt (DE)
(74) Representative: Kohol, Sonia

(57) **Abstract**

The present invention is a liquid handling member, with an inner region circumscribed and hermetically sealed by a wall region, which comprises a membrane assembly comprising a porous membrane material to separate a first zone outside of the member from a second zone within the member. The membrane has a first surface oriented towards the first zone, and a second surface towards the second zone, whereby the first zone is positioned in liquid communication with a liquid releasing source during its intended use, and the second zone is connected to a suction device. Thereby, the membrane assembly is capable of maintaining a pressure differential between the second zone and the first zone without permitting air to penetrate from said first zone to said second zone. Further, the first surface of the membrane comprises at least a first region and a second region, said regions differing in at least one physical surface property.

## Description

The present invention relates to membranes or membrane assemblies which can be used in hygiene articles or in other articles useful for handling or transporting or storing liquids, such as aqueous liquids like body fluids like urine or blood, but also for non-aqueous liquids such as oils. The present invention relates to membranes in such articles which have two distinct regions which exhibit at least one surface property differing between them.

### Specification/Description

Liquid handling systems comprising a membrane are known in the art. For example, US-A-5.678.564 discloses a liquid removal system designed to permit liquid removal through the use of an interface device. The interface device is provided with a membrane which has and is capable of maintaining a vacuum on one side so that when liquid contacts the opposite side of the membrane the liquid passes through the membrane and is removed from the interface device by a maintained vacuum to a receptacle for disposal. Such a system is described to be useful as a female external catheter system. Also, in PCT application US 98/13497 similar systems are disclosed.

It is further known from hygiene or personal care articles, that these can have varying surface properties on varying parts of the surface of the article. For example, baby diapers are known with varying hydrophilicities in various regions of the article, or with application of lotions or skin care compositions in certain regions of the topsheet.

However, hitherto such treatments have not been applied to membranes which are incorporated into liquid handling members.

### Summary

The present invention is a liquid handling member, with an inner region circumscribed and hermetically sealed by a wall region, which comprises a membrane assembly comprising a porous membrane material to separate a first zone outside of the member from a second zone within the member. The membrane has a first surface oriented towards the first zone, and a second surface towards the second zone, whereby the first zone is positioned in liquid communication with a liquid releasing source during its intended use, and the second zone is connected to a suction device. Thereby, the membrane assembly is capable of maintaining a pressure differential between the second zone and the first zone without permitting air to penetrate from said first zone to the second zone. Further, the first surface of the membrane comprises at least a first region and a second region, the regions differing in at least one physical property, which can be selected from the group of
- surface energy;
- open area of membrane pores;
- open pore size;
- surface roughness,
- coefficient of friction
- adhesion properties
- solubility for water or non-aqueous solvents;
- light refraction or color;
- gas / vapor adsorption properties;
- anti-microbial activity.

Preferably, the surface area of the first region is at least 10 times the area of a bubble point pressure defining pore of the membrane, and can be at least 0.25 mm².

The first zone can be treated with a surface property modifying agent, such as by applying a coating.

In particular executions, the first region can be treated with a hydrophobic surfactant, with a liquid barrier layer, or with skin care composition like a lotion.

In a further particular application, the first surface of the membrane is a three-dimensionally shaped, non-plane surface, such as being in the form of corrugations or pleats or folds, and the ridges of such corrugations can be treated with a surface property modifying agent, such as a skin care composition of the emollient or lotion type.

In a further aspect, the present invention relates to the process of modifying the properties of a membrane suitable for being used in liquid handling members by applying a surface property modifier selectively to first and second regions of the membrane.

### Detailed description

In the context of the present invention, a "liquid handling member" is considered to be a device, wherein a liquid is penetrating through a membrane by a driving force such as a suction like a vacuum. If this member is connected to a liquid delivery source, or liquid receiving sink, thus forming a "liquid handling system", this can be used in applications such as for - but without being limited to - receiving body liquids. In such applications, the liquid to be transported will be generally water based, such as body liquids like urine. It will be apparent to the skilled person, that the present invention is not limited to such applications, but that it can be readily re-applied to other liquids such as oily substances as disclosed in PCT applications US 99/14644 or US 99/14645.

Such systems function by the principle that certain porous membranes under certain conditions can be permeable to liquids, but not to gases like air, as long as the "potential differential " such as the pressure differential between the two sides of such a membrane does not exceed a certain value, which is characteristic for a given material and given liquid in the pores of the material - the "bubble point pressure". This latter is often expressed in "height of water column" which corresponds to the pressure exerted by such a column on the material under normal gravity conditions.

For aqueous liquids, the material for the membrane is preferably hydrophilic and has a pore size diameter on the order of about 5 to about 30 µm, more preferably about 10 to 20 µm. Once the membrane has been wetted it will support a suction pressure typically corresponding to about 12.5 cm to about 150 cm height of a water column without permitting air to pass therethrough. Thus, if suction is applied to one side of a wetted membrane, liquid contacting the membrane on the other side will be drawn by the suction through the membrane to the other side of the membrane, from where it can further be removed, for example by being sucked by means of a vacuum through a drain tube to a reservoir. As long as the filter material or membrane remains wet, air does not pass through the filter and suction is maintained without active pumping. If too much suction (too high vacuum) is applied to the membrane there is a risk that the bubble point of the membrane will be surpassed and there will be no liquid in one or more pores of the membrane, thereby allowing air or gas to penetrate through, which can lead to a loss of the vacuum, and of the liquid handling functionality. Thus the amount of vacuum should approach as close as possible - but not exceed - the bubble point.

Thereby, the membrane needs to maintain a certain degree of wetness, so as to maintain the pores filled with liquid, even under suction vacuum, and/or evaporation conditions. As described for example in US-A-5.678.564, the membrane pores can be prewetted during manufacture. This may be done by filling the pores with any suitable liquid having preferably a low vapor pressure. Glycerin has been proposed as a prewetting agent because it has a significant smaller propensity for drying out by evaporation and will generally support the vacuum until the first wetting during use.

A membrane material in the meaning of the present invention can be described by having in addition to the pore openings at least two distinctive surface regions, at least for the surface of the material, which is intended to be outside of the article, such as being oriented towards the user:

The pore openings - these relate to the ends of the pores through which the liquids can penetrate. When the pores in the membrane material do not have a constant diameter, e.g. considering a cross-section through such a pore having the shape of a cone, the wide part of the pore (respectively of the cone) can be considered to belong to the actual available surface, whilst the smaller parts of the cone, with diameters less than the one corresponding to the bubble point pressure, would be considered to belong to the "inner" surface of the membrane material. When not considering circular pore cross-section, the above discussion considers the cross-sectional equivalent diameter.

Further, the "land regions" , i.e. the remaining surface of the membrane. This land region can be described by comprising at least two distinctive regions, a first and a second membrane surface region. "Distinctive" refers to the fact, that these surface regions need to be sufficiently large to allow a meaningful assessment of their properties. In many cases, this will be readily achievable because the properties change from one sufficiently large region to the next sufficiently large region drastically, such as in a step change. In cases, where there are more gradual property changes, it will be apparent to the skilled person to classify the property level into at least two classes by appropriate averaging tools.

In either of the cases, the structures according to the present invention need to have sufficiently large regions to assess their properties. The minimal distinguishable surface areas are considered in the context of the present invention to be linked to the pore size of the membrane material, and should be at least ten times the equivalent area or the largest pores, i.e. the bubble point pressure determining pores, and often will be at least 0.25 mm.

The different regions can differ only by their surface properties, or can also have other differences, such as being distinct morphological. In particular, when such membranes do not have a flat surface (oriented towards the wearer during its intended use) i.e. have a 3-dimensionally shaped surface, such as described in co-pending application "Liquid handling systems comprising three-dimensionally shaped membranes" (co-filed with the present application under attorney docket number CM-2258FQ), it can be desired to have regions with different properties.

Membrane materials according to the present invention allow to address a number of contradicting requirements, such as the following by far not limiting explanatory examples.

For example, it is desirable to have a membrane with a high open area to allow high permeabilities (without compromising on maximum pore size requirements so as to not jeopardize membrane functionality i.e. not to unduly reduce bubble point pressure). However, such higher open areas carry the risk of not sufficiently masking undesirable discoloration's, such as of received body exudates, or of not satisfying overall certain mechanical strength requirements to withstand making, transporting, storing and use. In such cases, the open area can vary over the membrane surface (which thus would impact on the two land regions in a complementary manner).

More generally, the following surface properties of a membrane have been considered to be particularly suitable to provide distinct surface regions. These properties can be relevant for
either the functionality of the liquid handling member such as selective wetting,
or the interaction of the member with other elements of the article, such as providing regions with improved gluing properties;
or the interaction of the member with the user of the article comprising the member, such as by applying skin care compositions, or printing indicia such as for application of visual application or fit guides.

Whilst the benefits become particularly pronounced for properties with contradicting effects or requirements, also different degrees of the same effect are considered to fall with the scope of the invention, as long as the two regions can be determined with a sufficient statistical certainty (i.e. the effect should be bigger than the accuracy of the test method).

Whilst the present invention relates to different properties of the surface of the material only, also the underlying regions of the membrane may exhibit different properties. It should be noted, that - once applied to the membrane, a surface modifying coating would be considered to belong to the membrane material, even if it would increase the thickness of the membrane material in this particular region.

An important surface property relates to the surface energy of the material, also often related to hydrophilicity, hydrophobicity or oleophilicty, or oleophobicity as being depending on the contacting liquid.

Each material has a characteristic surface energy, as determined by the composition. For example, an extremely hydrophobic material, such as Teflon ® exhibits energies as low as 18 mN/m, polypropylene has a value of about 23 mN/m, and polyester materials have values in the range of 45 mN/m.

The surface energy of a material can be impacted by surfactants as well known in the art, which can be applied to the "bulk" of the membrane material (also referred to as resin incorporated surfactants) or these can be applied to the surface after the membrane material has been formed. The surfactant may be attached permanently to the material, or this can be done releasably, such as by having soluble or washable surfactants.

Surface energy can be determined by various methods, such as by placing drops of known surface tension on the surface and consider spreading or non-spreading of the liquid. A way to execute such tests is for example described in ASTM D-824, or D-5725.

The open area of the pores is considered a surface property in the context of the present invention.

For the membrane functionality, this is considered an important factor, as it impacts various liquid handling properties, such as bubble point pressure, or permeability.

The open area can be impacted by the pore size respectively pore size distribution, as well as the number of pores per unit are, as well as by the shape of the pores.

The open area can be determined such as by well known optical methods, such as by placing a sample structure under a suitable microscope, and determining the relevant parameter manually or by using commercially available computerized counter, such as by using a Coulter counter or by applying the teaching of ASTM E-1382 Linked to the open area is the porosity of a membrane material, such as can be determined according to ASTM E-1294.

A particular application of the present invention relates to a selective treatment of regions of the membrane which have, for example due to an undesired impact, holes or pores of a size, which are to large to maintain the liquid handling functionality of the member. In such instance, selective surface properties can be used to identify these spots, and to selectively apply means for closing these holes or pores.

A further important property of surfaces is the surface roughness, which relates to the coefficient of friction of the material to a reference material, such as polished steel, as can be determined by applying ASTM D-4103, or D-1894 for plastic film materials, respectively.

Selecting different properties in different regions can result in improved contact of the membrane material to other member materials (e.g. having a higher surface roughness to improve bonding) in regions contacting these other materials, whilst the surface in direct contact with the user exhibits a smooth surface. Such surface roughness can be assessed by ASTM F-1438, and is closely linked to the coefficient of friction, as can be measured by using ASTM D-4103 or D-1894 for plastic films respectively.

A particular effect of selective surface properties can be achieved by having membrane regions with varying degree of solubility to certain liquids, such as solvents or water based liquids. For example, in absorbent articles a first body liquid such as urine can dissolve a barrier layer, whilst the remainder of the membrane surface remains covered with such a barrier layer, even if contacted by a second body exudate such as fecal material. Generally, such solubilities can be determined by ASTM E-1184 for aqueous liquids, or D-3132 for non-aqueous ones.

Yet another effect can relate to regions of the membrane have varying adhesion properties, such as generally can be assessed by using ASTM D-2979, or D-6195.

For example, for absorbent articles, or external catheters, it can be desirable to have certain parts of the membrane to adhere to the skin of the wearer by being treated with body adhesives. As this coating can have detrimental effects to the liquid handling properties it can be desirable to have these only in limited regions. A more detailed desription of such applications can be found in co-pending application "Hygiene article comprising membrane containing interface device and body adhesives", Attorney docket number CM-22161FQ.

Further, the membrane material may have different light refraction effects or colors, such as can be determined by ASTM E-1541 or E-1331, respectively. This can have aesthetic effects, but also can be used to apply certain marking on the membrane to aid application of the article by a user. It also can have a masking effect in certain parts of the article.

Further, the membrane can have different surface adsorption properties with regard to adsorbing substances from the gas or vapor phase, such as odor. Typical measurements are described in ASTM F-726. The surface in one region can also have a different microbiological activity than other regions, such by being treated with anti-microbial agent, such as can be determined in analogy to ASTM D-3456.

The different surface properties of the regions of the membrane materials can be achieved by various ways.

For example, the membrane can be made by using materials with different properties, which are arranged in a particular manner so as to create the differing regions, such as by weaving threads of different properties in a certain, preferably repeating, pattern of sufficient size.

Alternatively, a membrane can be made from a homogeneous material, and the different surface properties are created by applying a certain treatment to a selected region, which can be an energy treatment (such as plasma or ion-beam treatment), or by mechanical treatment, such as selective stretching), or by chemical treatment. Often, such differing properties can be achieved by selective application of surface property modifying agents, such as surfactants or coatings.

Without wishing to limit applications of the present invention, the following describes applications to exemplify the benefits thereof.

When the membrane in an fluid handling member such as for application in an hygiene article is shaped in a three-dimensional shape (such as described in more detail in co-pending application "Liquid handling member comprising three-dimensionally shaped membranes", attorney docket number CM-2258FQ), skin care treatments or lotions such as described in EP-A-0794.804, can be selectively applied to the regions of the structure, which are in direct contact with the skin of the wearer.

Alternatively, the "upper" parts of the structure (i.e. the parts oriented towards the wearer during use) can be more hydrophobic than the lower parts, so as to improve the dryness for the skin of the wearer.

If such three-dimensional structures are shaped in the form of corrugations, or folds or pleats, application of re-enforcement resin to top of ridges can improve stability, collapse, and impact on the friction on top of the ridges.

## Claims

1. A liquid handling member, said member having a inner region circumscribed and hermetically sealed by a wall region, said wall region comprising a membrane assembly comprising a membrane material to separate a first zone
outside of the member from a second zone within said member,
said membrane having a first surface oriented towards the first zone, and a second surface towards the second zone,
whereby said first zone is positioned in liquid communication with a liquid releasing source during its intended use, and said second zone is connected to a suction device,
whereby said membrane assembly is capable of maintaining a pressure differential between the second zone and the first zone without permitting air to penetrate from said first zone to said second zone.
characterized in that
said first surface of the membrane comprises at least a first region and a second region, said regions differing in at least one physical property.

2. A liquid handling member according to claim 1, wherein said physical property is selected form the group of
surface energy;
open area of membrane pores;
open pore size;
surface roughness,
coefficient of friction
adhesion properties
solubility for water or non-aqueous solvents;
light refraction or color;
gas / vapor adsorption properties;
anti-microbial activity.

3. A liquid handling member according to claim 1 or 2, wherein said first region is at least 10 times the area of a bubble point pressure defining pore of the membrane.

4. A liquid handling member according any of claims 1 to 3, wherein said first region has a surface area of at least 0.25 mm².

5. A liquid handling member according to any of the preceding claims, wherein said first zone is treated with a surface property modifying agent.

6. A liquid handling member according to claim 5,wherein said treatment with a surface property modifying agent is a coating.

7. A liquid handling member according to any of claims 5 or 6, wherein said first region is treated with a hydrophobic surfactant.

8. A liquid handling member according to any of claims 5 to 7, wherein said first region is treated with a liquid barrier layer.

9. A liquid handling member according to any of the preceding claims, wherein said first region is treated with a skin care lotion.

10. A liquid handling member according to claim 9, wherein said lotion is capable of being transferred to the skin of a wearer during its intended use.

11. A liquid handling member according to claims any of the preceding claims, wherein said first surface of said membrane is a three-dimensionally shaped, non-plane, surface.

12. A liquid handling member according to claim 11, wherein said three-dimensional shape is in the form of corrugations or pleats or folds.

13. A liquid handling member according to claims 11 or 12, wherein said three-dimensionally shaped, non-flat surface has protrusions extending with an distal end into said first zone, whereby said first regions correspond to said distal ends.

14. An hygiene article comprising a liquid handling member according to any of the preceding claims.

15. Process of modifying the properties of a membrane suitable for being used in liquid handling members according to any of the preceding claims by applying a surface property modifier selectively to first and second regions of said membrane.
